# EUROPÄISCHE PATENTSCHRIFT

(11) **EP 2 616 116 B1**
(45) Veröffentlichungstag und Bekanntmachung des Hinweises auf die Patenterteilung: **03.12.2014**
(21) Anmeldenummer: 11743180.9
(22) Anmeldetag: 21.07.2011
(51) Int. Cl.: A61M 1/06, F04B 35/04

(54) **MEMBRANVAKUUMPUMPE**
MEMBRANE VACUUM PUMP
POMPE À VIDE À MEMBRANE

(30) Priorität: 17.09.2010 WO PCT/CH2010/000225; 17.09.2010 CH 15022010
(43) Veröffentlichungstag der Anmeldung: 24.07.2013
(73) Patentinhaber: Medela Holding AG, 6340 Baar (CH)
(72) Erfinder: FELBER, Armin, CH-6003 Luzern (CH); WEBER, Beda, CH-5643 Sins (CH); KOCH, Roland, CH-5620 Bremgarten (CH); FURRER, Etienne, CH-6300 Zug (CH)
(74) Vertreter: Clerc, Natalia
(86) Internationale Anmeldenummer: PCT/CH2011/000171
(87) Internationale Veröffentlichungsnummer: WO 2012/034238

(56) Entgegenhaltungen:
- EP-A1- 1 850 005
- WO-A1-99/44650
- DE-A1- 1 579 774
- GB-A- 2 465 797
- US-A- 5 644 177
- US-B1- 6 758 657

## Beschreibung

### TECHNISCHES GEBIET

Die vorliegende Erfindung betrifft eine Membranvakuumpumpe gemäss Oberbegriff des Patentanspruchs 1.

### STAND DER TECHNIK

Derartige Membranvakuumpumpen werden für verschiedene medizinaltechnische Anwendungen, insbesondere für Drainageapplikationen wie Wunddrainage oder Thoraxdrainage eingesetzt. Auch als Brustpumpen zum Abpumpen von menschlicher Muttermilch sind sie hinlänglich bekannt. Beispiele hierfür sind WO 96/22116, US 2009/0099511, US 2008/0287037, US 7 094 217 und US 2008/0039781.

Üblicherweise besteht der Antrieb aus einem Elektromotor, welcher über einen Exzenter, einem Pleuel oder einer sonstigen Kraftübertragungseinheit die Rotationsbewegung des Motors auf die Membran überträgt und diese zyklisch auslenkt. Nachteilig an vielen derartigen Pumpen ist, dass sie relativ gross und laut sind. Insbesondere bei sogenannten "hand-free" Anwendungen sollte die Pumpe jedoch möglichst diskret und somit möglichst klein und geräuscharm ausgebildet sein. "hands-free" bedeutet in diesem Zusammenhang, dass die gesamte Vorrichtung nach dem Einschalten ohne Hände funktioniert, d.h. dass weder die Pumpe noch die Brusthaube von Hand gehalten werden müssen.

WO 02/102437 und WO 2008/137678 zeigen beispielsweise "hands-free"-Abpumpvorrichtungen. Hier ist die Brusthaube jeweils in einem Pumpengehäuse integriert und dient gleichzeitig als Membran zur Erzeugung eines Unterdrucks. Als Antrieb ist jeweils ein Schrittmotor eingesetzt.

GB 2 465 797 offenbart eine zweiteilige Pumpe für ein Wunddrainagesystem. Ein Kolben eines elektromagnetischen Antriebs drückt auf eine Membran, wobei die Membran mit einer Rückstellfeder wieder in ihre Ausgangsposition bringbar ist.

WO 99/44650 offenbart einen Elektromotor mit einer Spindel, an welchem ein Kolben angeordnet ist. Der Kolben wirkt auf eine Vakuummembran.

EP 1 850 005 offenbart zwei Elektromagnete und einen dazwischen angeordneten Oszillator, welcher auf eine Vakuummembran wirkt.

### DARSTELLUNG DER ERFINDUNG

Es ist eine Aufgabe der Erfindung, eine Vakuumpumpe zu schaffen, welche möglichst klein ausgebildet ist.

Diese Aufgabe löst eine Vorrichtung mit den Merkmalen des Anspruchs 1.

Die erfindungsgemässe Membranvakuumpumpe weist ein elektrisch betriebenes Antriebsaggregat und eine Membran auf, welche eine Pumpkammer in einen antriebsseitigen Teil und einen antriebsfernen Teil trennt und welche mittels des Antriebsaggregats auslenkbar ist. Erfindungsgemäss ist das Antriebsaggregat ein elektromagnetisches Antriebsaggregat und die Auslenkung der Membran erfolgt in Richtung einer im Antriebsaggregat elektromagnetisch erzeugten linearen Bewegung.

Derartige Antriebsaggregate lassen sich sehr klein ausbilden. Dank der direkten Kopplung mit der Membran und der Auslenkung der Membran in dieselbe Richtung wie das elektromagnetisch bewegliche Teil des Aggregats, sind auch keine aufwendigen und platzverbrauchenden Kraftübertragungselemente notwendig.

Vorteilhaft ist ferner, dass diese lineare Bewegung im Vergleich zu den sonst üblichen rotierenden Antrieben von Membranvakuumpumpen ruhiger ist und weniger Vibrationen und Körperschall erzeugt werden. Die Hublänge lässt sich zudem im Gegensatz zu den bekannten Membranvakuumpumpen verändern. Sie kann insbesondere elektronisch gesteuert werden. Dies erlaubt eine präzise Regelung auch bei hohen Vakuumpegeln.

In einer bevorzugten Ausführungsform weist das Antriebsaggregat mindestens einen Permanentmagneten und einen Spulenkörper mit einer Spule, wobei die Spule auf dem Spulenkörper angeordnet ist, wobei der Spulenkörper mit der Spule bezüglich des Magneten entlang einer Längsachse linear verschiebbar gehalten ist und wobei der Spulenkörper mit der Membran verbunden ist und diese bei der genannten Verschiebung des Spulenkörpers in Richtung seiner Verschiebung auslenkt.

Der Spulenkörper weist vorzugsweise eine Länge auf, welche um ein Vielfaches grösser ist als seine Breite. Er kann ein- oder mehrstückig ausgebildet sein. Vorzugsweise weist die Membran einen um ein Vielfaches grösseren Durchmesser auf als der Kolben. Vorzugsweise ist die Membran im Wesentlichen kreisförmig ausgebildet und besteht aus einem dünnen Material, insbesondere aus Silikon.

Der Spulenkörper dient vorzugsweise als Kolben für die Auslenkung der Membran. Der Kolben weist ein erstes Ende auf, an welchem die Membran angeordnet ist. Die Kraftübertragung erfolgt somit sehr direkt und präzise. Die Auslenkung der Membran lässt sich gezielt steuern und die Vakuumpumpe arbeitet innerhalb einer sehr engen Toleranzgrenze. Die Präzision lässt sich noch erhöhen, wenn die Membran mittig am ersten Ende des Kolbens befestigt ist.

Typischerweise arbeitet die Vakuumpumpe bis zu einem Unterdruck von 0 bis 300 mmHg. Die Membran kann mit einer Frequenz von 5 bis 120 Zyklen pro Minute angetrieben werden.

Der Kolben weist ein zweites Ende auf, welches in einer ersten bevorzugten Ausführungsform linear verschieblich gehalten ist.

Alternativ kann an diesem zweiten Ende auch eine zweite Membran befestigt sein. Diese zweite Membran ist vorzugsweise identisch zur ersten Membran ausgebildet und ebenfalls in einer Pumpkammer angeordnet. Somit ist eine Doppelpumpe vorhanden, welche vorzugsweise mittig bezüglich ihrer Längsachse spiegelsymmetrisch ausgebildet ist.

In einer bevorzugten Ausführungsform ist der bewegliche Teil des Aggregats mit einem Belüftungsventil wirkverbunden, so dass die lineare Bewegung des beweglichen Teils des Aggregats wahlweise auch zugleich zur Betätigung des Belüftungsventils verwendet wird. In einer bevorzugten Ausführungsform ist das Belüftungsventil hierzu am der Vakuummembran gegenüberliegenden Ende des beweglichen Aggregatteils angeordnet. Das bewegliche Aggregatteil öffnet bei entsprechendem Hub das Belüftungsventil. Vorzugsweise weist das Aggregatteil hierzu mindestens einen Stift auf, welcher auf mindestens eine Ventilklappe einwirkt, so dass diese eine Belüftungsöffnung freigibt. Vorzugsweise ist die Ventilklappe Teil einer Belüftungsmembran, welche am beweglichen Aggregatteil befestigt ist.

Da das bewegliche Aggregatteil auch zur Betätigung des Belüftungsventils eingesetzt werden kann, ist der Aufbau des Aggregats vereinfacht, und es werden relativ wenig Einzelteile benötigt. Vorteilhaft ist, dass zur Betätigung des Belüftungsventils kein separater Motor und keine separate Steuerung notwendig sind. Dies erhöht die Prozesssicherheit und reduziert die Kosten.

Vorteilhaft ist ferner, dass beide Membranen, d.h. die Vakuummembran wie auch die Belüftungsmembran als Lagerung für den beweglichen Teil des Aggregats dienen, so dass Reibung vermieden wird. Dieses Pumpaggregat kann somit relativ lange wartungsfrei eingesetzt werden.

Die Spule ist in einem bevorzugten Ausführungsbeispiel, insbesondere in den oben genannten Beispielen, eine Flachspule und es sind mindestens zwei Permanentmagnete vorhanden, welche beidseits der Spule und des Spulenkörpers angeordnet sind. Vorzugsweise ist auf beiden Seiten der Spule je ein Permanentmagnetpaar vorhanden.

In einem anderen bevorzugten Ausführungsbeispiel ist das zweite Ende des Kolbens zwischen einem Eisenkern und einem Magneten verschieblich gelagert.

Die Spule kann eine Tauchspule sein, wobei die Tauchspule und der Permanentmagnet rotationssymmetrisch ausgebildet sind und die Tauchspule vom Eisenkern durchsetzt ist.

Vorzugsweise ist ein Positionsdetektor vorhanden zur Bestimmung der relativen Lage des Spulenkörpers zum Permanentmagneten. Dadurch lässt sich auch die jeweilige Auslenkung der Membran eindeutig bestimmen. Vorzugsweise ist der Positionsdetektor ein optischer Sensor. Auf dem Spulenkörper kann beispielsweise eine Positionsskala angeordnet sein, welche vom optischen Sensor überwacht wird. Die Positionsskala kann beispielsweise aus einer, zwei oder mehreren Graustufenbändern bestehen, welche sich mit dem Spulenkörper relativ zum Sensor bewegen.

Erzeugt der Positionsdetektor in Abhängigkeit der relativen Lage des Spulenkörpers ein Signal, welches zur Steuerung der Pumpe verwendet wird, so lässt sich die Auslenkung der Membran sehr präzise steuern. Die Vakuumpumpe arbeitete innerhalb eines sehr engen Toleranzbereiches in Bezug auf Frequenz und Amplitude, d.h. erzieltem Vakuum.

Diese Vakuumpumpe eignet sich für verschiedenste Anwendungsbereiche, insbesondere für den medizinaltechnischen Gebrauch. Eine bevorzugte Anwendung liegt im Abpumpen von menschlicher Muttermilch, also im Brustpumpenbereich. Weitere bevorzugte Anwendungen sind die Thoraxdrainage und die Wunddrainage.

In einer bevorzugten Brustpumpe, welche das erfindungsgemässe Aggregat aufweist, ist im antriebsfernen Teil der Pumpkammer ein Auslass vorhanden, welcher mit einem Einlass einer zweiten Kammer verbunden ist, wobei die Kammer eine zweite Membran aufweist, welche diese Kammer in zwei Teile trennt. Diese Membran dient zur Medientrennung und zur Übertragung des erzeugten Vakuums nach aussen. Dank dieser zweiten Membran lässt sich ein System einsetzen, welches von einem anfänglichen pneumatischen Unterdruck in einer an die Brust angelegten Brusthaube zu einem hydraulischen Unterdruck wechselt. Dabei wirkt bereits abgepumpte Milch als Fluid des hydraulischen Systems, welches weitere Milch aus der Brust abpumpt.

Die erfindungsgemässe Vakuumpumpe lässt sich jedoch auch für Brustpumpen einsetzten, welche ein zyklisches Vakuum an die Brusthaube anlegen und bei denen die Milch über einen von der Luftleitung getrennten Weg in einen Milchsammelbehälter gelangt.

Weitere vorteilhafte Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: einen Längsschnitt durch eine erfindungsgemässe Vakuumpumpe;
- Figur 2: einen Längsschnitt durch eine erfindungsgemässe Vakuumpumpe in einer zweiten Ausführungsform;
- Figur 3: eine Explosionsdarstellung einer Vorrichtung zum Abpumpen von Muttermilch mit der erfindungsgemässen Vakuumpumpe gemäss Figur 1;
- Figur 4: eine Vorrichtung zum Abpumpen von Muttermilch in einer zweiten Ausführungsform;
- Figur 5: eine Explosionsdarstellung einer erfindungsgemässen Vakuumpumpe in einer ersten Ausführungsform mit einem Teil der Vorrichtung gemäss Figur 3;
- Figur 6: einen Längsschnitt durch einen Teil der Vorrichtung gemäss Figur 5;
- Figur 7: eine schematische Darstellung einer erfindungsgemässen Vakuumpumpe in einer dritten Ausführungsform inklusive Steuerungseinheit;
- Figur 8: eine Explosionsdarstellung der Vakuumpumpe gemäss Figur 7;
- Figur 9: eine Seitenansicht der Vakuumpumpe gemäss Figur 7 in teilweise zusammengefügtem Zustand;
- Figur 10: einen Längsschnitt durch die Vakuumpumpe gemäss Figur 7 während der Vakuumerzeugung in einer ersten Position;
- Figur 11: einen vergrösserten Ausschnitt A gemäss Figur 10 mit einer geschlossenen Belüftungsklappe;
- Figur 12: einen vergrösserten Ausschnitt E gemäss Figur 10 mit einer geöffneten Auspuffklappe und geschlossener Vakuumklappe; Figur 13 einen Längsschnitt durch die Vakuumpumpe gemäss Figur 7 während der Vakuumerzeugung in einer zweiten Position;
- Figur 14: einen vergrösserten Ausschnitt D gemäss Figur 13 mit einer geschlossenen Auspuftklappe und geöffneter Vakuumklappe;
- Figur 15: einen Längsschnitt durch die Vakuumpumpe gemäss Figur 7 während der des Belüftens;
- Figur 16: einen vergrösserten Ausschnitt B gemäss Figur 15 mit einer geöffneten Belüftungsklappe und
- Figur 17: einen vergrösserten Ausschnitt C gemäss Figur 15.

Gleiche Teile sind in den Figuren mit denselben Bezugszeichen versehen.

### BESCHREIBUNG BEVORZUGTER AUSFÜHRUNGSFORMEN

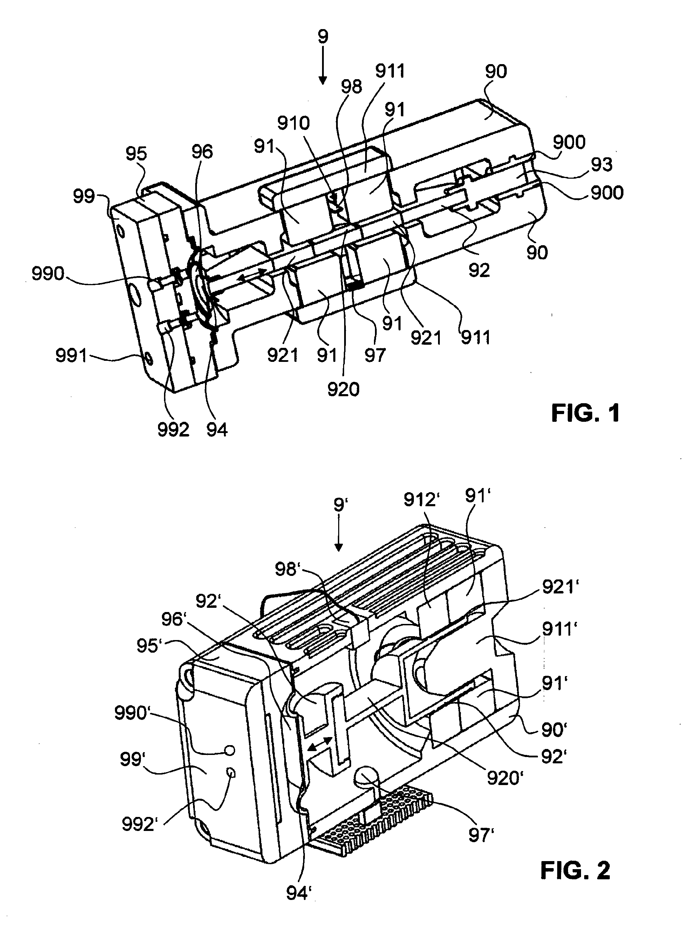

In Figur 1 ist eine erste Ausführungsform der erfindungsgemässen Vakuumpumpe bzw. eines erfindungsgemässen Pumpaggregats mit elektromagnetischem Antrieb dargestellt.

Das Pumpaggregat weist ein Gehäuse 90 auf, welches vorzugsweise aus Metall oder Kunststoff gefertigt ist. Das Gehäuse 90 weist vorzugsweise eine quaderförmige Gestalt auf. Im Gehäuse 90 ist auf einer Seite ein flacher Eisenkern bzw. eine Eisenplatte 911 und ein daran befestigter Dauermagnet 91 angeordnet. Die Eisenplatte 911 liegt auf dem Gehäuse 90 auf, so dass der Dauermagnet 91 in einer Ausnehmung des Gehäuses angeordnet ist. Der Dauermagnet 91 besteht aus zwei Teilen, welche beabstandet zueinander angeordnet sind, so dass zwischen ihnen eine Aussparung 910 vorhanden ist.

Auf einer gegenüberliegenden Seite des Gehäuses ist der identische Aufbau nochmals vorhanden. Auch hier sind eine Eisenplatte 911 und ein daran befestigter zweiteiliger Dauermagnet 91 angeordnet.

Zwischen diesen zwei einander gegenüberliegenden Dauermagnetpaaren 91 verläuft ein flacher Spulenkörper 92 mit einer in den Spulenkörper 92 eingelegten Spule 921.

Der Spulenkörper 92 ist im Wesentlichen stabförmig oder plattenförmig ausgebildet. An einem Ende ist er in einem Führungslager 93 lagefixiert gehalten. Das Führungslager 93 ist mit dem Spulenkörper 92 inklusive der Spule 921 relativ zum Gehäuse 90 und somit relativ zum Dauermagneten 91 entlang der Längsachse des Gehäuses 90 verschiebbar. Hierfür weist das Gehäuse 90 ein Gleitlager 900 auf. Die Bewegung des Spulenkörpers 92 ist in der Figur 1 mit einem Doppelpfeil dargestellt.

Das andere Ende des Spulenkörpers 92 ist fest mit einer Membran, hier Vakuummembran 94 genannt, verbunden. Die Vakuummembran 94 liegt am stirnseitigen Ende des Gehäuses 90 an diesem an und ist zwischen Gehäuse 90 und einer Ventilplatte 95 festgeklemmt. Die Membran 94 trennt eine Pumpkammer 96 vom Spulenkörper 92. Die Vakuummembran 94 weist vorzugsweise einen kreisförmigen Grundriss auf, wobei sie vorzugsweise eine für Membranen von Membranvakuumpumpen übliche Form aufweist. Die Ventilplatte 95 ist zwischen dem Gehäuse 90 und einem Deckel 99 gehalten. Vorzugsweise sind diese drei Teile lösbar oder unlösbar dicht miteinander verbunden, beispielsweise verschraubt. Die entsprechenden Löcher sind im Deckel 99 mit der Bezugsziffer 991 versehen. Der Deckel 99 weist eine Anschlussöffnung 990 für die Vakuumleitung 12' auf, welche mit der Pumpkammer 96 verbunden ist. Eine Lufteinlassöffnung 992 im Deckel 99 verbindet ebenfalls die Umwelt über die Ventilplatte 95 mit der Pumpkammer 96. Die Ventilplatte 95 weist die für Membranvakuumpumpen üblichen Ventile, Ein- und Auslässe auf. Diese werden hier nicht im Detail beschrieben.

Fliesst ein elektrischer Wechselstrom durch die Spule 921, so verändert sich das elektromagnetische Feld und der Spulenkörper 92 bewegt sich relativ zum Dauermagneten 91. Der Spulenkörper 92 wirkt wie ein Kolben oder Stössel und bewegt die Vakuummembran 94 zyklisch hin und her. Die Kraft, welche auf die Vakuummembran 94 wirkt, ist dabei proportional zu einem an die Spule angelegten Strom. Durch die Bewegung der Vakuummembran 94 wird in der Pumpkammer 96 ein sich zyklisch änderndes Vakuum aufgebaut, welches am Ausgang 990 anliegt.

Die Vakuummembran 94 des Pumpaggregats 9 wird somit über eine elektromagnetisch erzeugte lineare Bewegung angetrieben, wobei der Spulenkörper 92 als Kolben wirkt. Vorteilhaft ist, dass die Bewegung im Vergleich zu den sonst üblichen rotierenden Antrieben ruhiger ist und weniger Vibrationen und Körperschall erzeugt werden. Die Hublänge lässt sich im Gegensatz zum Stand der Technik verändern. Sie kann elektronisch gesteuert werden. Dies erlaubt eine präzise Regelung auch bei tiefen Vakuumpegeln.

Damit die Hublänge gezielt gesteuert werden kann, wird der Weg bzw. die Position des Spulenkörpers 92 überwacht. Hierfür werden Positions- und/oder Bewegungssensoren verwendet. In diesem Beispiel erfolgt dies durch einen optischen Sensor, welcher eine Positionsskala detektiert. Die Positionsskala 920 ist vorzugsweise auf dem Spulenkörper 92 aufgebracht. Eine Lichtquelle 97 sendet ihr Licht senkrecht zur Längsrichtung des Spulenkörpers 92 zu einem gegenüberliegenden Detektor 98, wobei es die Positionsskala 920 passiert. Der Spulenkörper 92 ist in diesem Bereich transparent ausgebildet.

Die Positionsskala kann beispielsweise durch einen, zwei oder mehrere Graustufenbalken gebildet sein, welche gemeinsam mit dem Spulenkörper den Sensor passieren. Sind zwei parallel zueinander verlaufende Graustufenbalken angeordnet, wobei einer eine ansteigende und einer eine abfallende Abstufung aufweist und werden zwei Sensoren verwendet, so kann die Differenz dieser zwei Signale als sehr präzise Positionsbestimmung verwendet werden.

Vorzugsweise sind die Lichtquelle 97 und der Detektor 98 in den Ausnehmungen 910 des Dauermagneten 91 angeordnet. Andere Arten der Positionsmessung sind möglich. Das gemessene Signal wird an eine elektronische Steuerung der Vakuumpumpe gesendet und der Strom wird nach Massgabe dieses Signals an die Spule angelegt. Dadurch lassen sich die Position, die Auslenkungsamplitude und die Frequenz unabhängig voneinander steuern. Es werden üblicherweise Vakuumwerte von 0 bis zu 300 mmHg erzielt. Die Frequenzen sind üblicherweise 0 bis 150 Zyklen pro Minute.

Alternativ kann anstelle des Führungslagers 93 auch eine zweite Membran angeordnet sein, welche ähnlich oder identisch zur Vakuummembran 94 ausgebildet ist. Dadurch ist ein symmetrischer Aufbau vorhanden, welcher ebenfalls eine Führung und somit eine lineare Bewegung des Spulenkörpers 92 innerhalb des Gehäuses 90 gewährleistet. Die zweite Membran kann zudem ebenfalls zum Aufbau des Vakuums verwendet werden, so dass die Flussrate gesteigert werden kann.

Figur 2 zeigt eine zweite Ausführungsform einer Vakuumpumpe mit einer elektromagnetisch erzeugten linearen Antriebsbewegung der Membran. Im Gegensatz zur oben beschriebenen Flachspule ist hier eine Tauchspule eingesetzt. Magnet und Spule sind rotationssymmetrisch, insbesondere ringförmig bzw. zylinderförmig ausgebildet. Auch hier ist ein Gehäuse 90' vorhanden. Ein Dauermagnet 91' ist am hinteren, der Brusthaube entfernten Ende der Tauchspule angeordnet. Er ist von einem Eisenkern 911' durchsetzt, welcher auf einer ersten Stirnseite des Dauermagneten 91' anliegt. Auf der gegenüberliegenden Stirnseite des Dauermagneten liegt ein Eisenring 912' an. Der Spulenkörper 92' umgreift den Eisenkern 911' und ist durch diesen axial geführt. Der Spulenkörper 92' erstreckt sich zwischen dem Eisenkern 911' und dem Dauermagneten 91' bzw. dem Eisenring 912'. Er ist im Dauermagneten 91' und im Eisenring 912' berührungslos geführt. Die umwickelte Spule 921' umgibt dabei den Spulenkörper 92' in diesem Bereich.

Auch hier ist der Spulenkörper 92' mit der Vakuummembran 94' fest verbunden und wirkt als Kolben für den linearen Antrieb der Vakuummembran 94'. Die Pumpkammer ist mit der Bezugsziffer 96', die Ventilplatte mit der Bezugsziffer 95' und der Deckel mit der Bezugsziffer 99' versehen. Die Anschlussöffnung weist die Bezugsziffer 990' und die Lufteinlassöffnung 992' auf. Auch hier ist wiederum ein Positionssensor vorhanden, welcher die Position des Spulenkörpers 92' relativ zum Magneten 91' und somit die Bewegung bzw. Position der Vakuummembran 94' an eine Steuerung meldet, um das Vakuum zu regulieren. Die Lichtquelle ist hier mit der Bezugziffer 97', der Detektor mit 98' und die Positionsskala mit 920' bezeichnet. Die Positionsskala ist vorzugsweise transparent ausgeführt. Im Gegensatz zum vorherigen Beispiel ist hier dieser Sensor ausserhalb des Bereichs des Dauermagneten 91' angeordnet.

Es werden in dieser Ausführungsform üblicherweise Vakuumwerte von 0 bis zu 300 mmHg erzielt. Die Frequenzen sind üblicherweise 0 bis 150 Zyklen pro Minute.

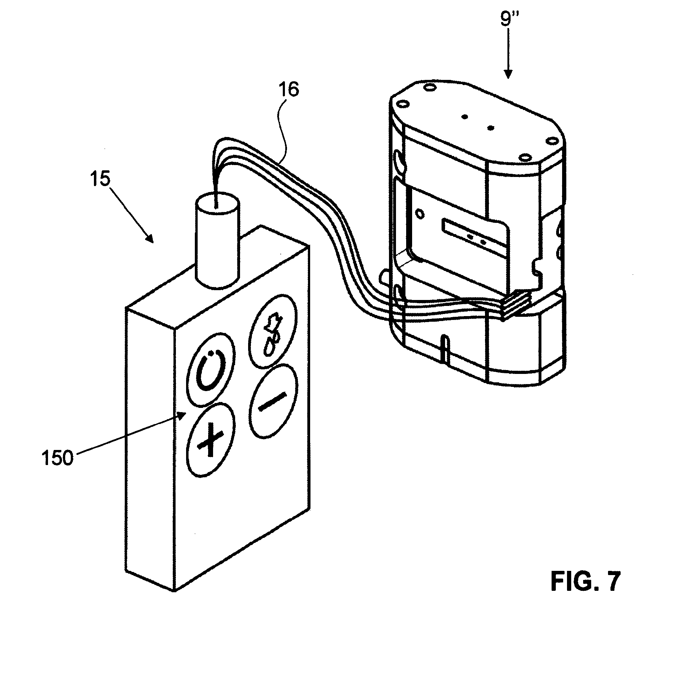

In den Figuren 7 bis 17 ist eine dritte Ausführungsform einer erfindungsgemässen Vakuumpumpe mit einer elektromagnetisch erzeugten linearen Antriebsbewegung der Membran dargestellt. Sie stellt im Wesentlichen eine Weiterentwicklung des Pumpaggregats gemäss Figur 1 dar.

Figur 7 zeigt das Pumpaggregat 9" gemäss der dritten Ausführungsform, welches über Leitungen 16 zur Stromversorgung und für den Datentransfer mit einer Steuereinheit 15 verbunden ist. Die Steuereinheit 15 verfügt über Bedienungselemente 150 zum Betrieb der Brustpumpe.

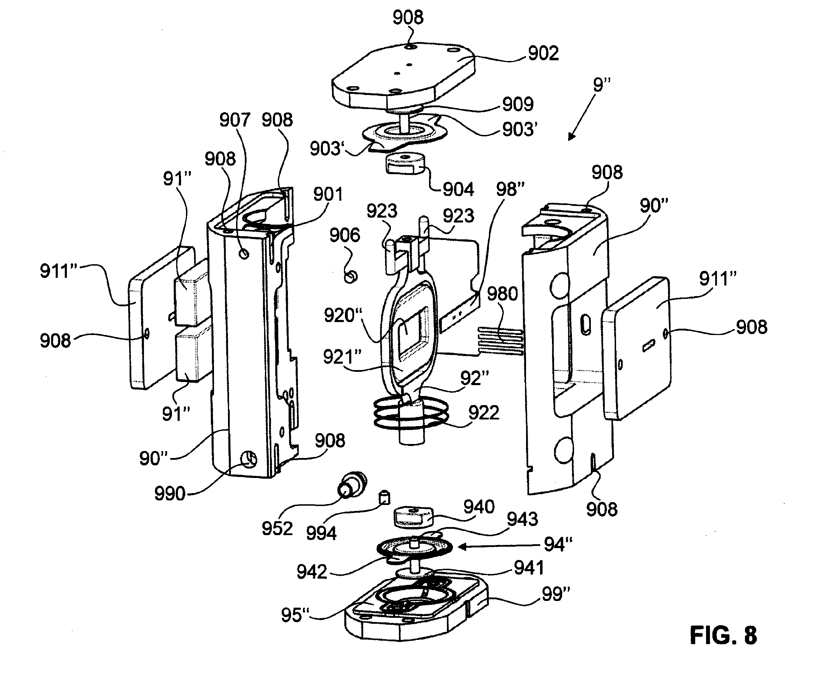

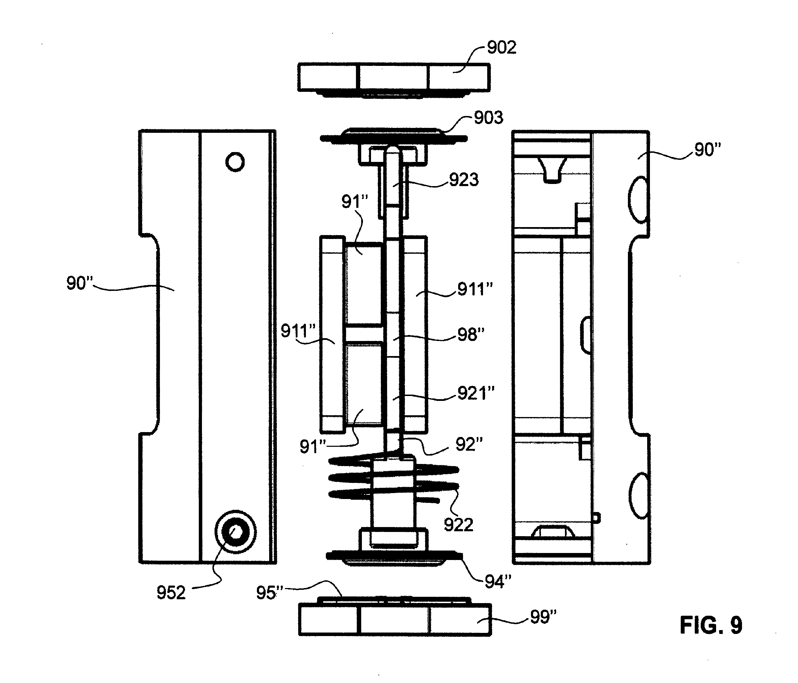

Figur 9 zeigt dieses Pumpaggregat 9" in einer teilweisen Explosionsdarstellung. Die Einzelteile des Aggregats 9" sind in Figur 8 gut erkennbar. Das Pumpaggregat 9" weist wiederum ein Gehäuse 90" auf, welches vorzugsweise aus Metall oder Kunststoff gefertigt ist. Das Gehäuse 90", gebildet durch zwei Seitenteile 90", ist auf beiden Seiten mit einem planen Deckel 99", 902 verschlossen. Die entsprechenden Befestigungslöcher 908 im Gehäuse 90" und in den Deckeln 99", 902 sind in Figur 8 gut erkennbar. Die zugehörigen Schrauben sind nicht dargestellt.

Im Gehäuse ist ein Spulenträger oder körper 92" angeordnet mit einer daran befestigten Flachspule 921". Endwindungen 922 der Spule 921" umgeben ein stabförmiges Ende des Spulenkörpers 92". Dieses stabförmige Ende weist vorzugsweise einen runden Querschnitt auf.

Die Spule 921" weist eine zentrale Ausnehmung auf, in welcher eine Positionsskala 920" angeordnet ist. Mittels eines gegenüberliegenden Senders und Empfängers bzw. Detektors 98" lässt sich anhand dieser Positionsskala 920" die Position des Spulenkörpers 92" relativ zum Gehäuse bestimmen. Der Sender/Empfänger 98" umfasst vorzugsweise eine lichtemittierende Diode und eine Photodiode. Die Positionsskala 920" kann beispielsweise eine Graustufenskala sein. Die Anschlüsse für einen zugehörigen Messprint sind in der Figur mit dem Bezugszeichen 980 bezeichnet. Andere Arten der Positionsbestimmung sind möglich.

Auf einer Seite des Spulenkörpers 92" ist eine Eisenplatte 911" angeordnet. Auf der anderen Seite sind Permanent- oder Dauermagnete 91" sowie eine daran anschliessende und die Dauermagnete 91" miteinander verbindende Eisenplatte 911" vorhanden. Die Dauermagnete 91" sind in dieser Ausführungsform vorzugsweise quaderförmig ausgebildet. Sie können auch wie in Figur 1 auf beiden Seiten der Spule 921" angeordnet sein. Ebenso können die Magnete 91 der Ausführungsform gemäss Figur 1 nur auf einer Seite der Spule vorhanden sein.

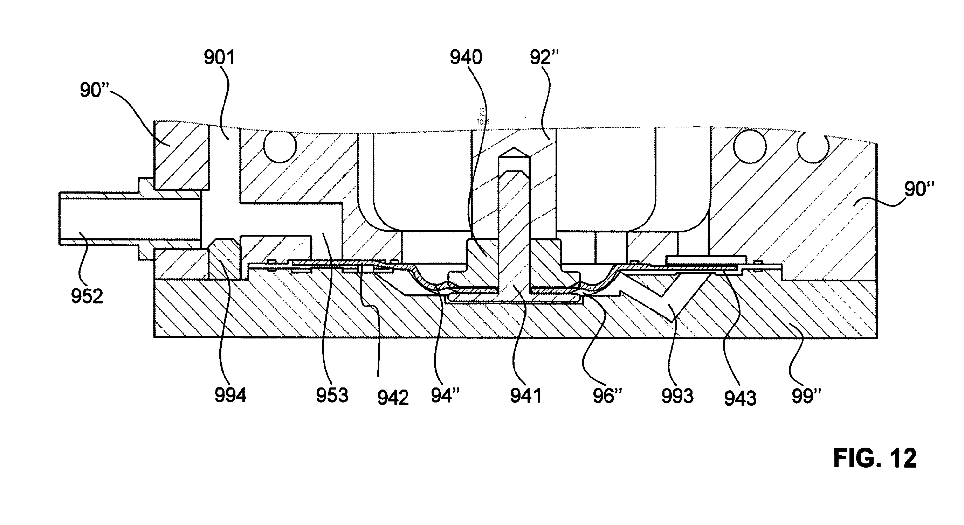

Auf einer Seite des länglichen Spulenkörpers 92" ist eine Vakuummembran 94" angeordnet und mit diesem fest verbunden. Die Vakuummembran 94" weist eine bekannte, vorzugsweise runde Form mit Rillen bzw. Sicken auf. Sie ist somit vorzugsweise im Wesentlichen tellerförmig. Sie ist vorzugsweise aus einem elastischen Material, insbesondere aus Silikon gefertigt. Zur Befestigung am Spulenkörper 92" ist die Membran mit einem zentralen Loch versehen, wie dies in Figur 12 erkennbar ist.

Die Vakuummembran 94" weist vorzugsweise zwei einander diametral entgegen gesetzte Flügel auf, welche einstückig mit der restlichen Membran 94" ausgebildet sind. Diese zwei Flügel bilden eine Einlassventil- oder Vakuumklappe 942 und eine Auslassventilklappe 943.

Die Vakuummembran 94" ist mit Klemmmitteln am ersten Ende des Spulenkörpers 92" befestigt. Hierfür sind ein erster Distanzhalter 940 und ein Gegenstück 941 vorhanden, wie dies in den Figuren 8 und 12 erkennbar ist. Das Gegenstück 941 weist hierfür einen Teller und einen daran angeformten Zapfen auf. Der erste Distanzhalter 940 ist quaderförmig mit einem Loch zur Aufnahme des Zapfens ausgebildet. Das Gegenstück 941 wird auf der Unterseite der Membran 94" angeordnet, der Zapfen durchdringt die Membran 94" und den ersten Distanzhalter 940 und greift in eine Öffnung im unteren Ende des Spulenhalters 92" ein. Vorzugsweise weisen der Zapfen und diese Öffnung je ein Gewinde auf.

Im Deckel 99" ist eine Ventilplatte 95" angeordnet. Sie kann in eine entsprechende Ausnehmung des Deckels 99" eingelegt sein oder einstückig in diesem ausgebildet sein. Die Ventilplatte 95" weist die für Membranpumpen üblichen Ventilöffnungen und -kanäle auf, welche mit den Ventilklappen 942, 943 in Verbindung stehen.

Ein Vakuumanschluss 952, welcher in den Figuren 8 und 12 gut erkennbar ist, führt nach aussen, so dass im Pumpaggregat, genauer in einer Pumpkammer 96"' erzeugtes Vakuum extern, z.B. in einer Brusthaube einer Brustpumpe, angelegt werden kann. Pumpkammer 96" und Vakuumanschluss 952 sind über einen Vakuumkanal 953 miteinander verbunden. Im Gehäuse 90" ist eine entsprechende Anschlussöffnung 990 vorhanden, um den Vakuumanschluss 952, hier ein Stutzen, nach aussen zu führen.

Erfindungsgemäss ist an dem der Vakuummembran 94" gegenüberliegenden Ende des Spulenkörpers 92" eine zweite Membran 903 angeordnet. Diese Membran 903 bildet mit dem rückseitigen Deckel 902 ein Ventil, welches zur Belüftung des Aggregats dient, genauer des Vakuumkanals 953 und somit des Vakuumanschlusses 952. Mit Hilfe dieser Belüftungsmembran 903 lässt sich ein extern angelegtes Vakuum schnell und gezielt reduzieren, so dass sich der Druck im Vakuumkanal 953 und am Vakuumanschluss 952 je nach Dauer der Belüftung auf Atmosphärendruck erhöht. Die Luft zur Belüftung stammt aus dem Innern des Gehäuses 90", welches ausser in den für das Erzeugen des Vakuums relevanten Bereichen gegen aussen nicht luftdicht verschlossen ist.

Diese Belüftungsmembran 903 ist ebenfalls fest mit dem Spulenkörper 92" verbunden. Sie ist vorzugsweise rund mit Rillen oder Sicken, d.h. tellerförmig ausgebildet, wobei auch sie zwei diametral gegenüberliegende Flügel aufweist, wobei mindestens einer davon, vorzugsweise beide, eine Belüftungsklappe 903' bildet. Die Belüftungsmembran 903 kann im Wesentlichen identisch ausgebildet sein wie die Vakuummembran 94". Sie kann jedoch auch eine andere Form und/oder Grösse aufweisen.

Die Belüftungsmembran 903 weist ein zentrales Loch auf, damit sie mit geeigneten Klemmmitteln am rückseitigen Deckel 902 befestigt werden kann. Die Klemmmittel sind vorzugsweise ein zweiter Distanzhalter 904 und ein Gegenstück 909. Dieser zweite Distanzhalter 904 und das Gegenstück 909 sind vorzugsweise gleich ausgebildet wie die entsprechenden Teile zur Befestigung der Vakuummembran 94". Auch hier ist ein Zapfen des Gegenstücks 909 in eine entsprechende Öffnung des zweiten Endes des Spulenkörpers 92" aufgenommen.

Dieses zweite Ende des Spulenkörpers 92" ist von mindestens einem, hier zwei Belüftungsaktuatoren 923 überragt. Die Belüftungsaktuatoren 923 sind in diesem Beispiel je ein zu den Belüftungsklappen 903' hin gerichteter, in axialer Richtung des Spulenkörpers 92' verlaufender Stift. Die Stifte 923 sind am Spulenkörper 92' angeordnet, vorzugsweise sind sie einstückig mit diesem ausgebildet. Sie sind einander diametral gegenüberliegend angeordnet. Der Abstand der Stifte 923 von der Mittelachse des Spulenkörpers 92' ist so bemessen, dass sie die Belüftungsklappen 903' berühren können. Es ist auch möglich, nur einen einzigen Stift zu verwenden. Aus Symmetriegründen, insbesondere um eine gleichmässige Bewegung des Spulenkörpers 92" zu ermöglichen, empfiehlt sich jedoch die Verwendung von zwei oder mehr Stiften.

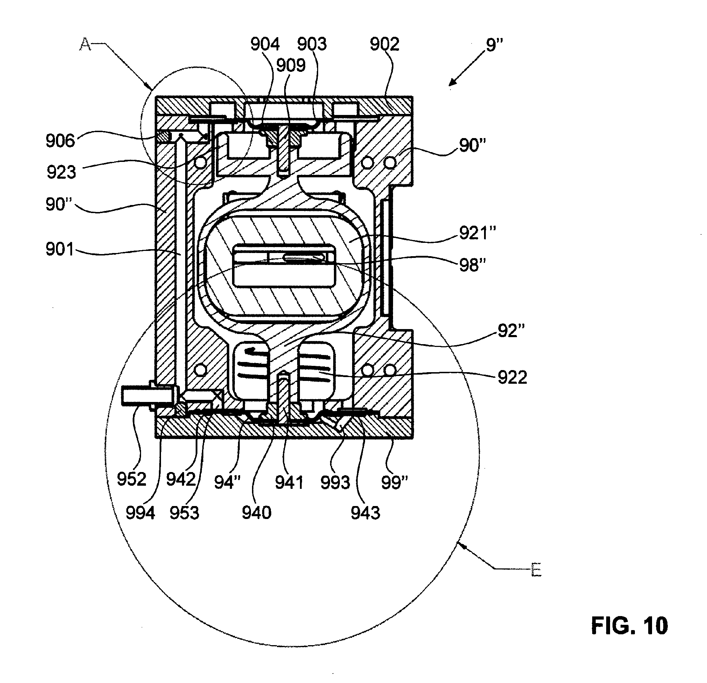

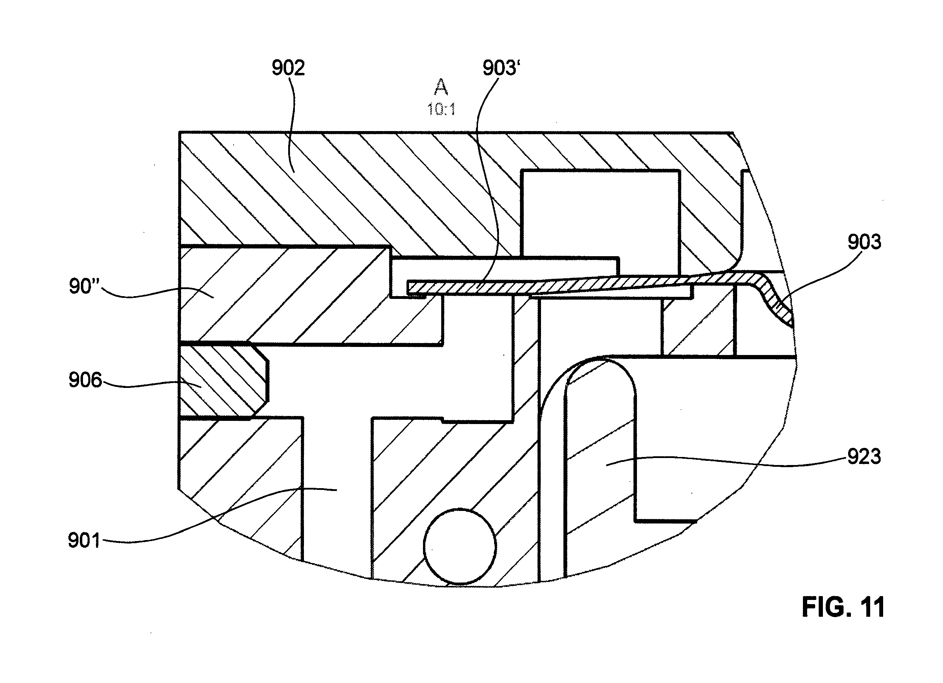

In Figur 10 ist ein Querschnitt durch das zusammengefügte Aggregat dargestellt. Es befindet sich in einer ersten maximalen Hubposition, in welcher ein Vakuum erzeugt wird. Der Spulenkörper 92" befindet sich in einer der Vakuumkammer 96" nahen Position, hier untere Position genannt. In Figur 11 ist erkennbar, dass die Belüftungsmembran 903 auf dem Gehäuse 90" aufliegt und die Belüftungsklappen 903' einen im Gehäuse 90" verlaufenden Belüftungskanal 901 verschliessen. Der Belüftungskanal 901 ist an einem Ende zwecks einfacherer Herstellung mit einem Verschlussstopfen 906 versehen, um eine entsprechende Bohrung 907 im Gehäuse 90" zu verschliessen. Aus demselben Grund ist das andere Ende des Belüftungskanals 901 ebenfalls mit einem Verschlussstopfen 994 versehen. Vorzugsweise ist die Belüftungsmembran 903 vorgespannt, um diesen Verschluss optimal zu gewährleisten.

Figur 12 zeigt die Situation im unteren, die Vakuummembran 94" beinhaltenden Bereich zu demselben Zeitpunkt. Die Vakuummembran 94" befindet sich in ihrer untersten Position, in welcher sie dem Deckel 99" am nächsten ist. Die Vakuumventilklappe 942 ist geschlossen, und die Verbindung zwischen Pumpkammer 96" und Vakuumkanal 953 ist unterbrochen. Die Auslassventilklappe 943 hingegen ist offen. Luft, welche sich noch im deckelseitigen Bereich der Pumpkammer 96" befand, wird dadurch über einen in der Ventilplatte 95' bzw. im Deckel 99" verlaufenden Auslasskanal 993 ins Gehäuse 90" bzw. nach aussen entlassen.

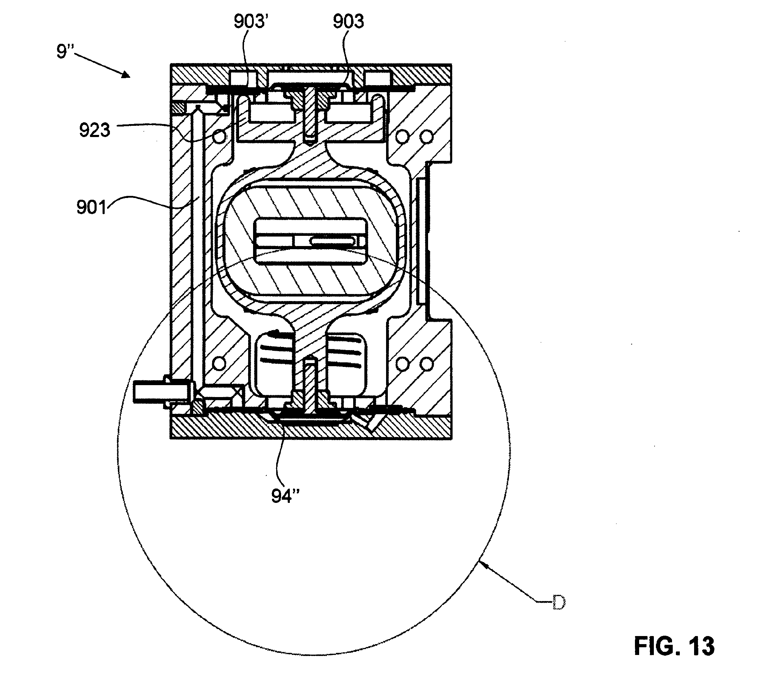

In Figur 13 ist der Spulenkörper 92" in einer zweiten Hubposition dargestellt. Die Belüftungsmembran 903 verschliesst nach wie vor den Belüftungskanal 901, so dass von aussen keine Luft in den Vakuumkanal 953 gelangen kann. Der Belüftungsaktuator 923 hat sich zwar der Belüftungsklappe 903' angenähert, sie jedoch noch nicht angehoben.

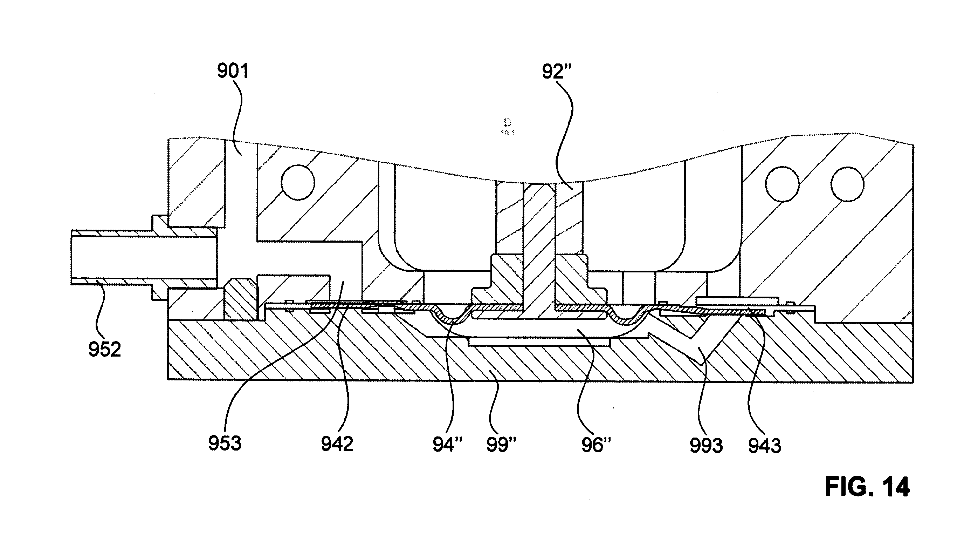

In Figur 14 ist diese Situation im Bereich der Vakuummembran 94" vergrössert dargestellt. Der deckelseitige Bereich der Vakuumkammer 96' ist dank der angehobenen Vakuummembran 94" expandiert und weist einen Unterdruck auf. Die Auslassventilklappe 943 ist geschlossen, die Vakuumklappe 942 hingegen offen, so dass der Unterdruck über den Vakuumkanal 953 an den Vakuumanschluss 952 angelegt wird. Der Belüftungskanal 901 bildet zwar ein zusätzliches Totvolumen, welches auch evakuiert werden muss. Da er jedoch ein relativ geringes Volumen aufweist, wirkt sich der Kanal 901 kaum störend auf die Leistungsfähigkeit des Pumpaggregats aus.

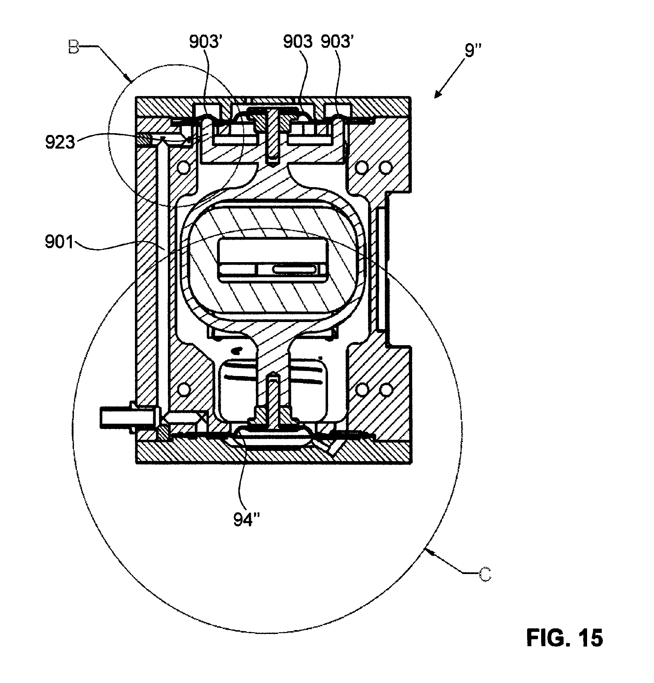

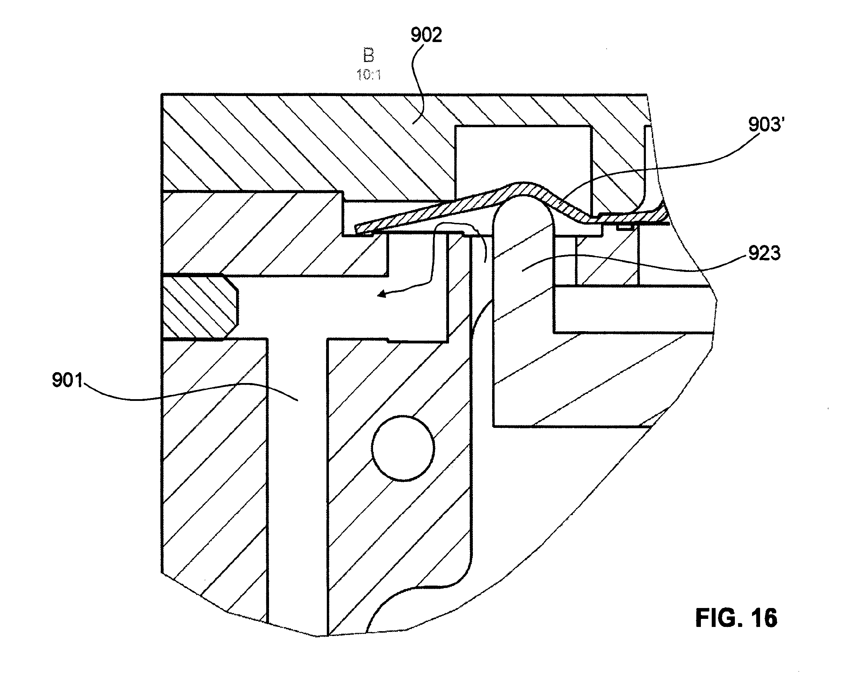

In Figur 15 ist der Spulenkörper 92" nun in einer dritten, noch weiter angehobenen Position dargestellt. In dieser Position heben die Aktuatoren 923 die Belüftungsklappen 903' an und öffnen so eine Verbindung zum Belüftungskanal 901. Luft kann vom Gehäuse 90" bzw. von aussen durch den Kanal 901 zum Vakuumanschluss 952 gelangen und so das angelegte Vakuum reduzieren und den Druck auf Atmosphärendruck oder auf ein gewünschtes Basisvakuum anheben. Dies ist in Figur 16 gut erkennbar. Der Pfeil zeigt den Weg der einströmenden Luft.

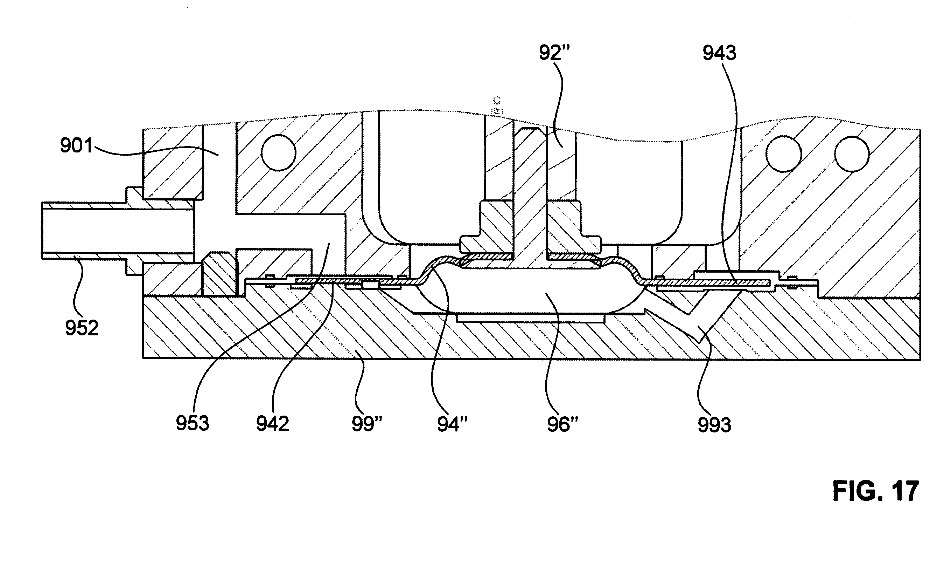

Wie in Figur 17 erkennbar ist, ist in dieser Situation die Vakuummembran 94" noch weiter angehoben und somit für den nächsten, vakuumerzeugenden Hub bereit. Die Vakuumklappe 942 und auch die Auslassklappe 943 sind beide angehoben und geben somit die entsprechenden Öffnungen frei.

In diesem erfindungsgemässen Pumpaggregat wird somit nicht nur die lineare Bewegung des Spulenkörpers 92" des Elektromagneten in eine parallele Bewegung der Vakuummembran umgesetzt. Es wird derselbe Spulenkörper 92" auch zum Öffnen des Belüftungsventils verwendet, wobei hierfür seine Bewegung in derselben Richtung wie für die Vakuumhübe ausgenützt wird. Je nach Position des Spulenkörpers 92" relativ zum Permanentmagnet 91" und somit zum Gehäuse 90" wird ein Vakuum erzeugt oder die Belüftung aktiviert. Hierzu sind lediglich drei verschiedene Hubpositionen des Spulenkörpers 92" notwendig. Der dritte Hub, welcher die Belüftung aktiviert, ist dabei grösser als jeder der zwei anderen Hübe oder er ist wenigstens grösser als der gleichgerichtete Hub zur Vakuumerzeugung. Diese drei Hübe sind, insbesondere in ihrer Grösse, kontrolliert und gezielt durch die elektronische Steuerung 15 erzeugbar.

Dieser dritte Hub wird, wie auch die zwei anderen Hübe, nach Massgabe der elektronischen Steuerung 15 des Aggregats 9" erzeugt. Der dritte Hub muss jedoch nicht in jedem Zyklus erfolgen. Dadurch lassen sich Pumpsequenzen mit unterschiedlichen und variierenden Kurvenformen und Pausen generieren.

Dank der Positionsskala lässt sich die Position des Spulenkörpers 92" relativ zum Gehäuse 90" genau erkennen und zur Steuerung der Hübe einsetzen. Andere Arten, um die Position des Spulenkörpers 92" und die Grösse der Hübe genau zu erkennen, sind jedoch auch möglich.

Vorteilhaft ist ferner, dass beide Membrane, d.h. die Vakuummembran 94" und die Belüftungsmembran 903 als Lager für die Bewegung des Spulenkörpers 92" dienen und somit Reibung vermieden wird.

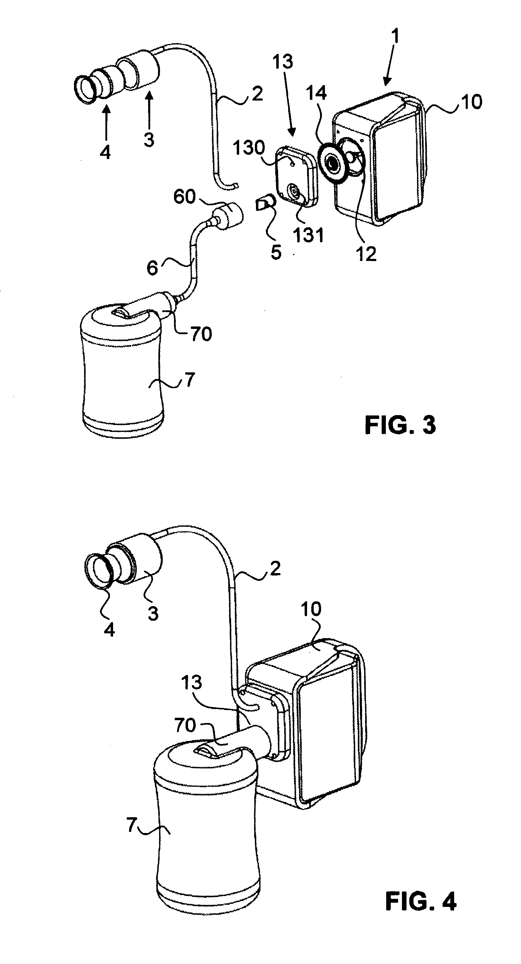

Die gemäss den oben beschriebenen Ausführungsbeispielen dargelegte erfindungsgemässe Vakuumpumpe lässt sich nun beispielsweise in Vorrichtungen zum Abpumpen von menschlicher Muttermilch einsetzen. Beispiele derartiger Vorrichtungen sind in den Figuren 3 bis 6 dargestellt.

In Figur 3 ist eine erste Ausführungsform einer derartigen Vorrichtung dargestellt. Sie weist eine Brustpumpe 1, eine erste Leitung 2, ein Kopplungsteil 3, eine Brusthaube 4, ein Rückschlagventil 5, eine zweite Leitung 6 und einen Milchsammelbehälter 7 auf.

Die Brusthaube 4 ist über das Kopplungsteil 3 und die erste flexible Leitung 2 mit der Brustpumpe 1 verbunden. Von der Brustpumpe 1 führt die zweite flexible Leitung 6 zum Milchsammelbehälter 7, wobei diese Verbindung mit dem Rückschlagventil 5 versehen ist.

Die zwei flexiblen Leitungen 2, 6 sind vorzugsweise Schläuche, insbesondere aus Silikon.

Wie in Figur 4 dargestellt ist, kann alternativ der Milchsammelbehälter 7 auch direkt an der Brustpumpe 1 befestigt sein. Hierfür ist vorzugsweise ein geeignet geformter Verbindungsstutzen 70 am Milchsammelbehälter 7 vorhanden, welcher mit einem Gehäuse 10 der Brustpumpe lösbar verbindbar ist.

Die Brustpumpe 1 weist das genannte Gehäuse 10 auf, in welchem die erfindungsgemässe Vakuumpumpe, nachfolgend Pumpaggregat genannt, gemeinsam mit einer Steuerung angeordnet ist. Das Pumpaggregat und die Steuerung können netzwerkbetrieben oder batteriebetrieben sein.

Am Gehäuse 10 sind hier nicht dargestellte Bedienungselemente vorhanden. Die Bedienungselemente können unter anderem einen Ein/Ausschaltelement, Tasten oder Knöpfe zur Wahl der Pumpfrequenz und des angelegten Vakuums und der Zeitdauer des Abpumpvorgangs enthalten. Es kann auch ein Display vorhanden sein.

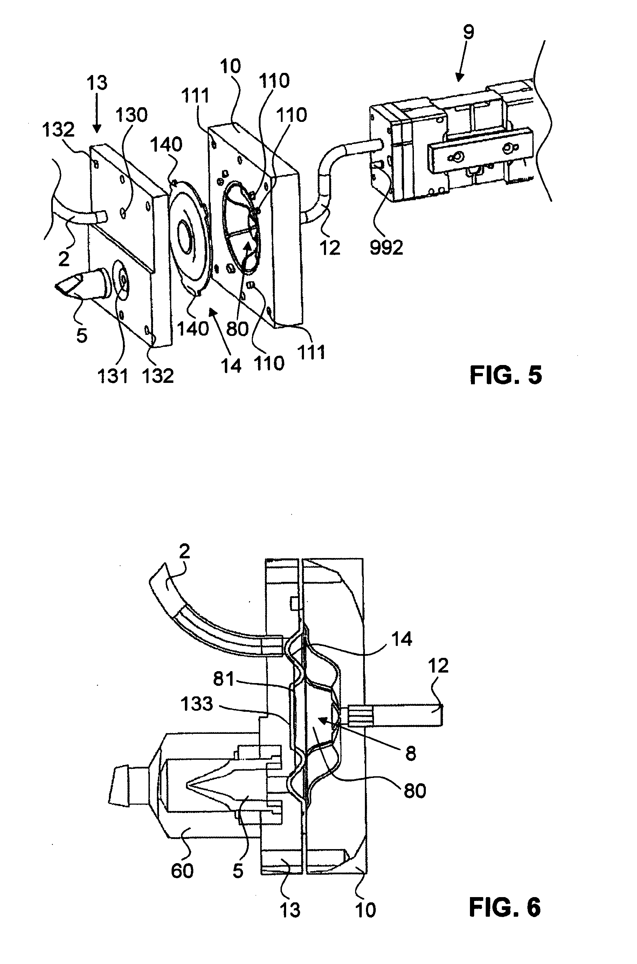

Zwischen einer Ausnehmung des Gehäuses 10 und einem diese Ausnehmung überdeckenden Deckel 13 ist eine zweite Kammer 8 (siehe Figur 6) gebildet, welche ebenfalls aus Pumpkammer wirkt. Der Deckel 13 ist vorzugsweise lösbar mit dem Gehäuse 10 verbunden. In dieser zweiten Kammer 8 ist eine Membran 14 angeordnet, welcher vom Deckel 13 in ihrer Lage gehalten ist. Die Membran 14 unterteilt diese Pumpkammer 8 in einen aggregatseitigen und einen aggregatfernen Teil 80, 81, wobei sie diese zwei Teile gegenseitig dichtet.

Die hier dargestellte Membran 14 weist, wie in Figur 5 erkennbar ist, vorzugsweise einen im Wesentlichen kreisförmigen Grundriss auf. Vorzugsweise sind seitlich vorstehende Flügel 140 vorhanden. Hier sind drei Flügel 140 vorhanden. Die Grundplatte, welche vorzugsweise Teil des Gehäuses 10 ist, weist seitliche Anschläge 110 auf, zwischen welchen diese Flügel 140 gehalten sind. Dadurch lässt sich die Membran 14 in eindeutiger Lage in der zweiten Kammer 8 halten. Dies erleichtert die Montage.

Der Deckel 13 weist Anschlüsse für die erste und zweite Leitung 2, 6 bzw. für den Milchsammelbehälter auf. Diese Anschlüsse sind mit den Bezugszeichen 130 und 131 versehen. Der zweite Anschluss 131 ist vorzugsweise mit einem Rückschlagventil 5 versehen. Die Verbindung mit der Gegenplatte, welche Teil der Gehäuses 10 ist, erfolgt vorzugsweise über Schnapp- oder Schraubverbindungen, wobei die entsprechenden Löcher in der Figur 5 mit den Bezugszeichen 132 und 111 versehen sind.

Vakuum, welches im Pumpaggregat erzeugt wurde, wird über eine Leitung 12 an diese zweite Kammer 8 übermittelt. Die Leitung 12 ist am Ausgang 990 angeschlossen. Die Druckänderungen werden über die Vakuumleitung 12 an die zweite Kammer 8 weitergeben, wo die Membran 14 analog zur ersten Membran 94 bewegt wird.

Die Pumpe lässt sich dabei mit zeitlich gleichbleibendem Zyklus betreiben oder die Saugkurve kann, wie dies im Stand der Technik bekannt ist, in ihrer Form, Frequenz und Intensität, dem Saugverhalten des Säuglings und/oder den Bedürfnissen der Mutter angepasst sein.

Die zweite Kammer 8 weist Ein- und Ausgänge auf, welche in den Figuren nicht alle sichtbar sind. Der Deckel 13 kann einstückig oder mehrstückig ausgebildet sein. Er bildet nicht nur einen dichten Abschluss sondern dient auch als Ventilplatte für die diese zweite Pumpkammer 8. Im Deckel 13 sind deshalb hier nicht im Detail dargestellte Kanäle und Ventile angeordnet, welche einen Aufbau des Vakuums im aggregatfemen, d.h. brustseitigen Teil 81 der zweiten Pumpkammer ermöglichen.

Im Deckel 13 ist die erste Auslassöffnung 130 vorhanden, welche die Umwelt mit dem deckelseitigen 81 Teil der Pumpkammer 8 verbindet. Diese Auslassöffnung 130 dient als erster Anschluss für die erste Leitung 2. Die zweite Auslassöffnung 131, welche ebenfalls den deckel- bzw. brustseitigen Teil 81 der zweiten Pumpkammer 8 mit der Umgebung verbindet, ist als zweiter Anschluss ausgebildet. Dieser zweite Anschluss ist mit dem Rückschlagventil 5 versehen. Hier wird ein Schnabelventil verwendet, welches auf einen Stutzen aufgesteckt ist. Andere Ventilarten sind jedoch auch einsetzbar.

Wird die Vorrichtung nun verwendet, so wird die Brusthaube 4 auf die Mutterbrust aufgesetzt, so dass sie mindestens die Brustwarze umfasst. Vorzugsweise ist zusätzlich maximal die Areola von der Brusthaube 4 umfasst. Die Brustpumpe 1 wird eingeschaltet und wie oben beschrieben betrieben. Das an die zweite Pumpkammer 8 übermittelte Vakuum evakuiert die erste Leitung 2, so dass in der Brusthaube 4 ein Unterdruck vorliegt. Dadurch wird Milch aus der Mutterbrust abgepumpt und gelangt durch die Brusthaube 4 und das Kopplungsteil 3 in die erste Leitung 2. Die Milch fliesst durch den ersten Anschluss 130 in den deckelseitigen Teil der zweiten Pumpkammer. Die abgepumpte Milch verlässt die zweite Pumpkammer 8 durch den zweiten Anschluss 131 und das Rückschlagventil 5 und gelangt über die zweite Leitung 6 (siehe Figur 3) bzw. je nach Ausführungsform auch direkt in den Milchsammelbehälter 7 (siehe Figur 4). Es ist somit keine separate Leitung für den Milchtransport vorhanden. Die erste Leitung 2 dient gleichzeitig als Saugleitung und als Milchtransportleitung. Die Vorrichtung wechselt somit nach anfänglichem pneumatischen Pumpen in ein hydraulisches Pumpen. Dies ist eine weitere Annäherung an das natürliche Saugen von Säuglingen.

Die Membran 14 in der zweiten Pumpkammer weist zwei Funktionen auf. Erstens dient sie als Scheidewand zwischen der Luft im pumpenseitigen Teil der zweiten Pumpkammer und der Milch im deckelseitigen Teil der zweiten Pumpkammer. Sie dient somit als Medientrennung. Dadurch verhindert sie, dass Milch in die Vakuumleitung 12 und somit in das Pumpaggregat gelangen kann. Sie verhindert aber auch, dass Verschmutzungen des Pumpaggregats in die erste und zweite Leitung 2, 6 gelangen können. Zweitens führt ihre zyklische Bewegung innerhalb der zweiten Pumpkammer dazu, dass sie die Milch fördert und transportiert. Dank dieser zweiten Funktion können während des Abpumpens Milchsammelbehälter 7, Brusthaube 4 und Brustpumpe 1 in voneinander unabhängigen Lagen angeordnet sein. Beispielsweise kann sich der Milchsammelbehälter 7 oberhalb der Brustpumpe 1 und/oder der Brusthaube 4 befinden. Auch die Brustpumpe 1 kann oberhalb des Milchsammelbehälters 7 und/oder der Brusthaube 4 sein. Dies ermöglicht der Mutter auch liegend abzupumpen oder, wenn sie sitzt, den Milchsammelbehälter 7 und die Brustpumpe 1 ausser Reichweite von Kleinkindern auf ein Regal oder eine andere erhöhte Plattform zu stellen.

Das Rückschlagventil 5 öffnet sich vorzugsweise erst bei genügendem Druck, d.h. wenn die zweite Pumpkammer 8 ausreichend mit Milch gefüllt ist. Dadurch lässt sich das Totvolumen, welches evakuiert werden muss, minimal halten.

Das Totvolumen lässt sich zudem reduzieren, indem eine kleine Brusthaube 4 verwendet wird, welche nur die Brustwarze und keinen oder einen möglichst geringen Teil der restlichen Brust umfasst. Eine geeignete Brusthaube 4 ist in den Figuren dargestellt. Alternative Formen von Brusthauben lassen sich auch einsetzen.

Die Elemente der oben beschriebenen Ausführungsformen lassen sich einzeln oder in Gruppen miteinander kombinieren, um weitere Ausführungsformen zu bilden.

Die erfindungsgemässe Vakuumpumpe ist relativ klein und kompakt und arbeitet geräuscharm. Sie eignet sich insbesondere für "hands-free", d.h. handfreie Anwendungen von Brustpumpen.

### BEZUGSZEICHENLISTE

| | | | |
|---|---|---|---|
| 1 | Brustpumpe | | Kammerteil |
| 10 | Gehäuse | 81 | brusthaubenseitiger |
| 110 | seitlicher Anschlag | | Kammerteil |
| 111 | Loch | | |
| 12 | Vakuumleitung | 9,9', 9" | Pumpaggregat |
| 13 | Deckel | 90, 90', 90" | Gehäuse |
| 130 | erster Anschluss | 900 | Gleitlager |
| 131 | zweiter Anschluss | 901 | Belüftungskanal |
| 132 | Loch | 902 | rückseitiger Deckel |
| 14 | Membran | 903 | Belüftungsmembran |
| 140 | seitlicher Flügel | 903' | Belüftungsklappe |
| 15 | Steuereinheit | 904 | zweiter Distanzhalter |
| 150 | Bedienungselemente | 906 | Verschlussstopfen |
| 16 | Leitungen | 907 | Bohrung |
| | | 908 | Befestigungslöcher |
| 2 | erste Leitung | 909 | zweites Gegenstück |
| | | 91, 91', 91" | Dauermagnet |
| 3 | Kopplungsteil | 910 | Ausnehmung |
| | | 911' | Eisenkern |
| 4 | Brusthaube | 911,911" | Eisenplatte |
| | | 912' | Eisenring |
| 5 | Rückschlagventil | 92, 92', 92" | Spulenkörper mit Spule |
| | | 920, 920' | Positionsskala |
| 6 | zweite Leitung | 920" | Positionsskala |
| 60 | Ventilkappe | 921, 921' | Spule |
| | | 921" | Spule |
| 7 | Milchsammelbehälter | 922 | Windungsenden |
| 70 | Verbindungsstutzen | 923 | Belüftungsaktuator |
| | | 93 | Führungslager |
| 8 | Pumpkammer | 94, 94', 94" | Vakuummembran |
| 80 | antriebsseitiger | 940 | erster Distanzhalter |
| 941 | erstes Gegenstück | 98, 98', 98" | Detektor |
| 942 | Vakuumventilklappe (Einlassventilklappe) | 980 | Anschluss an Messprint |
| | | 99, 99', 99" | Deckel |
| 943 | Auslassventilklappe | 990 | Anschlussöffnung |
| 95, 95', 95" | Ventilplatte | 991 | Loch |
| 952 | Vakuumanschluss | 992 | Lufteinlassöffnung |
| 953 | Vakuumkanal | 993 | Auslasskanal |
| 96, 96', 96" | Pumpkammer | 994 | Verschlussstopfen |
| 97, 97' | Lichtquelle | | |

## Patentansprüche

1. Membranvakuumpumpe mit einem elektrisch betriebenen Antriebsaggregat und einer Vakuummembran (94, 94', 94"), welche eine Pumpkammer (96, 96', 96") in einen antriebsseitigen Teil und einen antriebsfernen Teil trennt und welche mittels eines beweglichen Teils (92, 92', 92") des Antriebsaggregats auslenkbar ist, wobei das Antriebsaggregat ein elektromagnetisches Antriebsaggregat ist, wobei die Bewegung des beweglichen Teils (92, 92', 92") eine lineare Bewegung ist und wobei die Auslenkung der Vakuummembran (94, 94', 94") in Richtung dieser im Antriebsaggregat elektromagnetisch erzeugten linearen Bewegung erfolgt, **dadurch gekennzeichnet, dass** der bewegliche Teil (92") mit einem Belüftungsventil (903) zur Belüftung der Membranvakuumpumpe wirkverbunden ist, dass die im Antriebsaggregat erzeugte lineare Bewegung wahlweise das Belüftungsventil (903) betätigt und dass der bewegliche Teil (92") mittels einer Steuerung in einem ersten und zweiten Hub zur Erzeugung eines Vakuums in der Pumpkammer (96") bewegbar ist und in einem dritten Hub zur Betätigung des Belüftungsventils (903) bewegbar ist, wobei der dritte Hub in Richtung des zweiten Hubs erfolgt, jedoch grösser als dieser zweite Hub ist.

2. Membranvakuumpumpe nach Anspruch 1, wobei das bewegliche Teil (92") ein erstes Ende und ein zweites dem ersten Ende gegenüberliegendes Ende aufweist, wobei die Vakuummembran (94") mit dem ersten Ende wirkverbunden ist und das Belüftungsventil (903) mit dem zweiten Ende wirkverbunden ist.

3. Membranvakuumpumpe nach einem der Ansprüche 1 oder 2, wobei das Belüftungsventil eine Belüftungsmembran (903) aufweist.

4. Membranvakuumpumpe gemäss Anspruch 3, wobei die Vakuummembran (94") fest mit dem ersten Ende und die Belüftungsmembran (903) fest mit dem zweiten Ende des beweglichen Teils (92") verbunden sind und dass sie die Lagerung des beweglichen Teils (92") innerhalb des Antriebsaggregats bilden.

5. Membranvakuumpumpe nach einem der Ansprüche 1 bis 4, wobei das Antriebsaggregat mindestens einen Permanentmagneten (91, 91', 91 ") und einen Spulenkörper (92, 92', 92") mit einer Spule aufweist, wobei die Spule auf dem Spulenkörper (92, 92', 92") angeordnet ist, wobei der Spulenkörper (92, 92', 92") mit der Spule bezüglich des Magneten (91, 91', 91") entlang einer Längsachse in zwei Richtungen linear verschiebbar gehalten ist und wobei der Spulenkörper (92, 92', 92") den beweglichen Teil bildet und fest mit der Vakuummembran (94, 94', 94") verbunden ist und diese bei der genannten Verschiebung des Spulenkörpers (92, 92', 92") in beiden Richtungen seiner Verschiebung auslenkt.

6. Membranvakuumpumpe nach Anspruch 5, wobei der Spulenkörper (92, 92', 92") einen Kolben mit einem ersten Ende bildet, an welchem die Vakuummembran (94, 94', 94") angeordnet ist.

7. Membranvakuumpumpe nach Anspruch 6, wobei die Vakuummembran (94, 94', 94") einen um ein Vielfaches grösseren Durchmesser als der Kolben (92, 92', 92") aufweist.

8. Membranvakuumpumpe nach Anspruch 7, wobei die Vakuummembran (94, 94', 94") mittig auf dem ersten Ende des Kolbens (92, 92', 92") befestigt ist.

9. Membranvakuumpumpe nach einem der Ansprüche 6 bis 8, wobei der Kolben (92, 92', 92") ein zweites Ende aufweist, welches linear verschieblich gehalten ist.

10. Membranvakuumpumpe nach einem der Ansprüche 6 bis 9, wobei der Kolben (92, 92") ein zweites Ende aufweist, an welchem eine weitere Membran (903) befestigt ist.

11. Membranvakuumpumpe nach einem der Ansprüche 6 bis 10, wobei die Spule (921, 921") eine Flachspule ist und wobei mindestens ein Permanentmagnet (91, 91") vorhanden ist, welcher lagefixiert in einem Gehäuse (90, 90") der Mebranvakuumpumpe gehalten ist.

12. Membranvakuumpumpe nach einem der Ansprüche 6 bis 9, wobei der Kolben (92') ein zweites Ende aufweist, welches zwischen einem Eisenkern (911') und einem Magneten (91') verschieblich gelagert ist.

13. Membranvakuumpumpe nach einem der Ansprüche 6 bis 9 oder 12, wobei die Spule eine Tauchspule ist, die Tauchspule und der Permanentmagnet rotationssymmetrisch ausgebildet (91') sind und die Tauchspule von einem Eisenkern (911') durchsetzt ist.

14. Membranvakuumpumpe nach einem der Ansprüche 1 bis 13, wobei mindestens ein Positionsdetektor (97, 97'; 98, 98', 98") vorhanden ist zur Bestimmung der relativen Lage des beweglichen Teils (92, 92', 92") des Antriebsaggregats zum lagefixierten restlichen Teil des Antriebsaggregats.

15. Membranvakuumpumpe nach Anspruch 14, wobei der Positionsdetektor (97, 97'; 98, 98', 98") in Abhängigkeit der relativen Lage des Spulenkörpers (92, 92', 92") ein Signal erzeugt, welches zur Steuerung der Vakuumpumpe verwendet wird.

16. Membranvakuumpumpe nach einem der Ansprüche 1 bis 15, wobei im antriebsfernen Teil der Pumpkammer (96, 96', 96") ein Auslass (990) vorhanden ist, welcher mit einem Einlass einer zweiten Kammer (8) verbunden ist, wobei die zweite Kammer (8) eine weitere Membran (14) aufweist, welche diese zweite Kammer (8) in zwei Teile (80, 81) trennt und wobei die weitere Membran (14) als Medientrennung und zur Übertragung des in der Pumpkammer erzeugten Vakuums dient.

## Claims

1. A diaphragm vacuum pump with an electrically operated drive unit and a vacuum diaphragm (94, 94' 94"), which separates a pump chamber (96, 96', 96") into a drive-side part and a drive-remote part and which can be deflected by means of a movable part (92, 92', 92") of the drive unit, wherein the drive unit is an electromagnetic drive unit, wherein the movement of the movable part (92, 92', 92") is a linear movement and wherein the vacuum diaphragm (94, 94', 94") is deflected in the direction of said linear movement which is generated electromagnetically in the drive unit, **characterized in that** the movable part (92") is operatively connected to a ventilation valve (903) for ventilating the diaphragm vacuum pump, wherein the linear movement generated in the drive unit optionally actuates the ventilation valve (903) and **in that** the movable part (92") is movable by means of a controller in a first and second stroke in order to generate a vacuum in the pump chamber (96") and is movable in a third stroke in order to actuate the ventilation valve (903), wherein the third stroke takes place in the direction of the second stroke but is larger than said second stroke.

2. The diaphragm vacuum pump as claimed in claim 1, wherein the movable part (92") has a first end and a second end opposite the first end, wherein the vacuum diaphragm (94") is operatively connected to the first end, and the ventilation valve (903) is operatively connected to the second end.

3. The diaphragm vacuum pump as claimed in claim 1 or 2, wherein the ventilation valve has a ventilation diaphragm (903).

4. The diaphragm vacuum pump as claimed in claim 3, wherein the vacuum diaphragm (94") is connected fixedly to the first end of the movable part (92") and the ventilation diaphragm (903) is connected fixedly to the second end thereof, and wherein said diaphragms form the mounting of the movable part (92") within the drive unit.

5. The diaphragm vacuum pump as claimed in one of claims 1 to 4, wherein the drive unit has at least one permanent magnet (91, 91', 91 ") and a coil former (92, 92', 92") with a coil, wherein the coil is arranged on the coil former (92, 92', 92"), wherein the coil former (92, 92', 92") together with the coil is held in a linearly displaceable manner in two directions along a longitudinal axis with respect to the magnet (91, 91', 91"), and wherein the coil former (92, 92', 92") forms the movable part and is fixedly connected to the vacuum diaphragm (94, 94', 94") and deflects the latter during the abovementioned displacement of the coil former (92, 92', 92") in both directions of the displacement of said coil former.

6. The diaphragm vacuum pump as claimed in claim 5, wherein the coil former (92, 92', 92") forms a piston with a first end at which the vacuum diaphragm (94, 94', 94") is arranged.

7. The diaphragm vacuum pump as claimed in claim 6, wherein the vacuum diaphragm (94, 94', 94") has a diameter that is much larger than the piston (92, 92', 92").

8. The diaphragm vacuum pump as claimed in claim 7, wherein the vacuum diaphragm (94, 94', 94") is fastened centrally on the first end of the piston (92, 92', 92").

9. The diaphragm vacuum pump as claimed in one of claims 6 to 8, wherein the piston (92, 92', 92") has a second end which is held in a linearly displaceable manner.

10. The diaphragm vacuum pump as claimed in one of claims 6 to 9, wherein the piston (92, 92") has a second end to which a further diaphragm (903) is fastened.

11. The diaphragm vacuum pump as claimed in one of claims 6 to 10, wherein the coil (921, 921 ") is a flat coil and wherein there is at least one permanent magnet (91, 91 "), which is held in a fixed position in a housing (90, 90") of the diaphragm vacuum pump.

12. The diaphragm vacuum pump as claimed in one of claims 6 to 9, wherein the piston (92') has a second end, which is mounted displaceably between an iron core (911') and a magnet (91').

13. The diaphragm vacuum pump as claimed in one of claims 6 to 9 or 12, wherein the coil is a moving coil, the moving coil and the permanent magnet (91') are of rotationally symmetrical design, and an iron core (911') passes through the moving coil.

14. The diaphragm vacuum pump as claimed in one of claims 1 to 13, wherein there is at least one position detector (97, 97'; 98, 98', 98") for determining the relative position of the movable part (92, 92', 92") of the drive unit with respect to the remaining part of the drive unit which is in a fixed position.

15. The diaphragm vacuum pump as claimed in claim 14, wherein the position detector (97, 97'; 98, 98', 98") generates a signal, which is used to control the vacuum pump, as a function of the relative position of the coil former (92, 92', 92").

16. The diaphragm vacuum pump as claimed in one of claims 1 to 15, wherein there is an outlet (990) in the drive-remote part of the pump chamber (96, 96', 96"), said outlet being connected to an inlet of a second chamber (8), wherein the second chamber (8) has a further diaphragm (14), which separates said second chamber (8) into two parts (80, 81) and wherein the further diaphragm (14) serves as a means of separating media and for transferring the vacuum generated in the pump chamber.

## Revendications

1. Pompe à vide à membrane comprenant un ensemble d'entraînement à commande électrique et une membrane de vide (94, 94', 94"), laquelle sépare une chambre de pompe (96, 96', 96") en une partie côté entraînement et une partie éloignée de l'entraînement, et laquelle peut être déviée au moyen d'une partie mobile (92, 92', 92") de l'ensemble d'entraînement, l'ensemble d'entraînement étant un ensemble d'éntraînement électromagnétique, le mouvement de la partie mobile (92, 92', 92") étant un mouvement linéaire et la déviation de la membrane de vide (94, 94', 94") s'effectuant dans la direction de ce mouvement linéaire généré de manière électromagnétique dans l'ensemble d'entraînement, **caractérisée en ce que** la partie mobile (92") est en liaison fonctionnelle avec une soupape d'aération (903) pour l'aération de la pompe à vide à membrane, **en ce que** le mouvement linéaire généré dans l'ensemble d'entraînement actionne de manière sélective la soupape d'aération (903), et **en ce que** la partie mobile (92") peut être déplacée au moyen d'une commande suivant une première et une deuxième course pour générer un vide dans la chambre de pompe (96") et peut être déplacée suivant une troisième course pour l'actionnement de la soupape d'aération (903), la troisième course s'effectuant dans la direction de la deuxième course mais étant plus grande que cette deuxième course.

2. Pompe à vide à membrane selon la revendication 1, dans laquelle la partie mobile (92") présente une première extrémité et une deuxième extrémité opposée à la première extrémité, la membrane de vide (94") étant en liaison fonctionnelle avec la première extrémité et la soupape d'aération (903) étant en liaison fonctionnelle avec la deuxième extrémité.

3. Pompe à vide à membrane selon la revendication 1 ou 2, dans laquelle la soupape d'aération comprend une membrane d'aération (903).

4. Pompe à vide à membrane selon la revendication 3, dans laquelle la membrane de vide (94") est reliée solidement à la première extrémité et la membrane d'aération (903) est reliée solidement à la deuxième extrémité de la partie mobile (92"), et celles-ci forment le support de la partie mobile (92") à l'intérieur de l'ensemble d'entraînement.

5. Pompe à vide à membrane selon l'une quelconque des revendications 1 à 4, dans laquelle l'ensemble d'entraînement comprend au moins un aimant permanent (91, 91', 91") et un corps de bobine (92, 92', 92") présentant une bobine, la bobine étant disposée sur le corps de bobine (92, 92', 92"), le corps de bobine (92, 92', 92") présentant la bobine étant maintenu de manière mobile linéairement dans deux sens le long d'un axe longitudinal par rapport à l'aimant (91, 91', 91"), et le corps de bobine (92, 92', 92") formant la partie mobile et étant relié solidement à la membrane de vide (94, 94', 94") et déviant celle-ci lors dudit déplacement du corps de bobine (92, 92', 92") dans les deux sens de son déplacement.

6. Pompe à vide à membrane selon la revendication 5, dans laquelle le corps de bobine (92, 92', 92") forme un piston présentant une première extrémité sur laquelle est disposée la membrane de vide (94, 94', 94'').

7. Pompe à vide à membrane selon la revendication 6, dans laquelle la membrane de vide (94, 94', 94") présente un diamètre plusieurs fois supérieur à celui du piston (92, 92', 92").

8. Pompe à vide à membrane selon la revendication 7, dans laquelle la membrane de vide (94, 94', 94") est fixée centralement sur la première extrémité du piston (92, 92', 92").

9. Pompe à vide à membrane selon l'une quelconque des revendications 6 à 8, dans laquelle le piston (92, 92', 92") présente une deuxième extrémité qui est maintenue de manière déplaçable linéairement.

10. Pompe à vide à membrane selon l'une quelconque des revendications 6 à 9, dans laquelle le piston (92, 92") présente une deuxième extrémité à laquelle est fixée une membrane supplémentaire (903).

11. Pompe à vide à membrane selon l'une quelconque des revendications 6 à 10, dans laquelle la bobine (921, 921") est une bobine plate, et dans laquelle au moins un aimant permanent (91, 91") est présent, lequel est maintenu de manière fixée en position dans un boîtier (90, 90") de la pompe à vide à membrane.

12. Pompe à vide à membrane selon l'une quelconque des revendications 6 à 9, dans laquelle le piston (92') présente une deuxième extrémité, laquelle est montée mobile entre un noyau de fer (911') et un aimant (91').

13. Pompe à vide à membrane selon l'une quelconque des revendications 6 à 9 ou 12, dans laquelle la bobine est une bobine mobile, la bobine mobile et l'aimant permanent étant réalisés de manière à présenter une symétrie de révolution (91') et la bobine mobile étant traversée par un noyau de fer (911').

14. Pompe à vide à membrane selon l'une quelconque des revendications 1 à 13, dans laquelle au moins un détecteur de position (97, 97'; 98, 98', 98") est présent pour déterminer la position relative de la partie mobile (92, 92', 92") de l'ensemble d'entraînement par rapport à la partie restante, fixée en position, de l'ensemble d'entraînement.

15. Pompe à vide à membrane selon la revendication 14, dans laquelle le détecteur de position (97, 97'; 98, 98' , 98") génère un signal en fonction de la position relative du corps de bobine (92, 92', 92"), lequel signal est utilisé pour la commande de la pompe à vide.

16. Pompe à vide à membrane selon l'une quelconque des revendications 1 à 15, dans laquelle une sortie (990) est présente dans la partie éloignée de l'entraînement de la chambre de pompe (96, 96', 96"), laquelle sortie est reliée à une entrée d'une deuxième chambre (8), dans laquelle la deuxième chambre (8) comprend une membrane supplémentaire (14), laquelle sépare cette deuxième chambre (8) en deux parties (80, 81), et dans laquelle la membrane supplémentaire (14) sert de séparation de milieux et sert à la transmission du vide généré dans la chambre de pompe.
